(19) Europäisches Patentamt
European Patent Office
Office européen des brevets

(11) Publication number: **0 003 278**
**A1**

# (12) EUROPEAN PATENT APPLICATION

(21) Application number: 79100015.1

(22) Date of filing: 04.01.79

(51) Int. Cl.[2]: **C 07 D 213/64**
C 07 D 213/85, C 07 D 213/80
C 07 D 401/04, C 07 D 221/04
C 07 D 217/26, C 07 D 217/24
A 61 K 31/44, A 61 K 31/435
//C07D213/61, C07D213/73

(30) Priority: 04.01.78 US 866961

(43) Date of publication of application:
08.08.79 Bulletin 79/16

(84) Designated contracting states:
BE CH DE FR GB LU NL SE

(71) Applicant: Merck & Co., Inc.
Patent Department 126 East Lincoln Avenue
Rahway, N.J. 07065(US)

(72) Inventor: Baldwin, John James
1680 Wagon Wheel Lane
Lansdale Pa. 19446(US)

(72) Inventor: Ponticello, Gerald Salvatore
937 Crest Road
Lansdale Pa. 19446(US)

(74) Representative: Abitz, Walter, Dr.-Ing. et al,
Abitz, Morf, Gritschneder P.O.Box 860109
D-8000 München 86(DE)

## (54) Polysubstituted-2-(3-loweralkylamino-2-R0-propoxy)pyridines, process for preparing the same, and pharmaceutical compositions containing the same.

(57) Novel substituted (3-loweralkylamino-2-$R_1$O-propoxy) pyridines, their pharmaceutically acceptable salts and formula

I

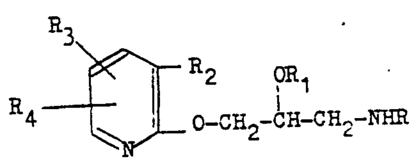

or

II

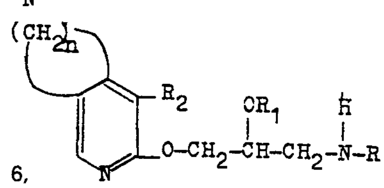

wherein
n is 3, 4, 5 or 6,
R is $C_3$-$C_4$ branched alkyl,
$R_1$ is H or $-\overset{O}{\overset{\|}{C}}-L$ wherein L is selected from $C_1$-$C_{10}$ alkyl, phenyl and substituted phenyl having up to two substituents which are independently selected from $C_1$-$C_4$ alkyl, $C_1$-$C_4$ alkoxy and halo,
$R_2$ is F, CN, $CF_3$, Cl, 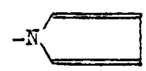 , $NO_2$,
-$COOR_5$ wherein $R_5$ is H, $C_1$-$C_6$ alkyl or $C_6$-$C_{12}$ carbocyclic aryl,
-$CONR_6R_7$ wherein $R_6$ and $R_7$ when separate, are H or $C_1$-$C_6$ alkyl and when joined, are $-CH_2-(CH_2)_3-CH_2-$, $-CH_2-CH_2-O-CH_2-CH_2$, $-CH_2-CH_2-NH-CH_2-CH_2-$ or $-CH_2-CH_2-N(CH_3)-CH_2-CH_2-$,
and
$R_3$ and $R_4$ are independently selected from H, $C_1$-$C_4$ alkyl, $CF_3$, F, 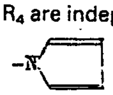 ,
$C_1$-$C_4$alkyl-$\overset{O}{\overset{\|}{C}}$-NH, phenyl and substituted phenyl wherein said substituents are independently selected from $C_1$-$C_4$alkyl, $C_1$-$C_4$alkoxy and halo, such that one of $R_3$ and $R_4$ is other than H,
and pharmaceutically acceptable salts thereof, and their preparation are disclosed. These pyridines have pharmaceutically useful properties such as β-adrenergic blocking activity.

EP 0 003 278 A1

0003278

Title

Polysubstituted-2-(3-loweralkylamino-2-$R_1$O-propoxy)pyridines, process for preparing the same, and pharmaceutical compositions containing the same

## Background of the Invention

The present invention concerns substituted (3-loweralkylamino-2-$R_1$O-propoxy)pyridines having pharmaceutically useful properties.

β-Adrenergic blocking agents or β-blockers are known to be useful in treating certain cardiovascular disorders such as arrhythmia, angina pectoris. While these β-blockers can also have antihypertensive activity, the onset of this activity is generally gradual. The structure and activity of β-blockers is generally discussed in "Clinical Pharmacology and Therapeutics" 10, 252, 306 (1969). Substituted N-heteroaryl β-adrenergic blocking agents are disclosed in British patent 1,305,644, U.S. 4,000,282, U.S. 3,946,009, Journal of Medicinal Chemistry 16, 1113-1114 (1973) and Journal of Medicinal Chemistry 15, 1321 (1972).

Novel substituted (3-loweralkylamino-2-$R_1$O-propoxy)pyridines have been discovered. These compounds have β-adrenergic blocking activity; some compounds also have antihypertensive activity of immediate onset.

## Summary of the Invention

Novel substituted (3-loweralkylamino-2-$R_1$O-propoxy)pyridines and their pharmaceutically acceptable salts which have β-adrenergic blocking activity.

## Description of the Preferred Embodiments

An embodiment of the present invention is compounds having the formula

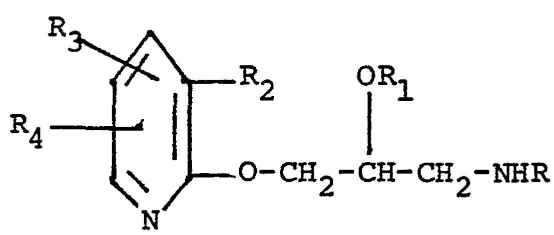

I

or

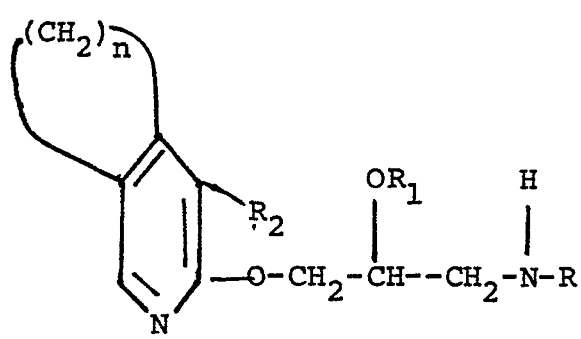

II

wherein

0003278

n is 3, 4, 5 or 6,

R is $C_3$-$C_4$ branched alkyl,

$R_1$ is H or $-\overset{O}{\overset{\|}{C}}-L$ wherein L is selected from $C_1$-$C_{10}$ alkyl, phenyl and substituted phenyl having up to two substituents which are independently selected from $C_1$-$C_4$ alkyl, $C_1$-$C_4$ alkoxy and halo,

$R_2$ is F, CN, $CF_3$, Cl, 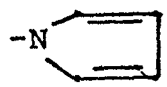 $NO_2$,

$-COOR_5$ wherein $R_5$ is H, $C_1$-$C_6$alkyl or $C_6$-$C_{12}$ carbocyclic aryl,

$-CONR_6R_7$ wherein $R_6$ and $R_7$ when separate, are H or $C_1$-$C_6$alkyl and when joined, are $-CH_2-(CH_2)_3-CH_2$, $-CH_2-CH_2-O-CH_2-CH_2-$, $-CH_2-CH_2-NH-CH_2-CH_2-$, or $-CH_2-CH_2-N(CH_3)-CH_2-CH_2-$,

and

$R_3$ and $R_4$ are independently selected from H, $C_1$-$C_4$alkyl, $CF_3$, F, 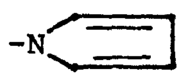 , phenyl, substituted phenyl having up to two substituents which are independently selected from $C_1$-$C_4$ alkyl, $C_1$-$C_4$alkoxy and halo, or $C_1$-$C_4$alkyl 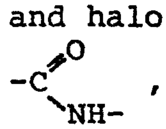 , such that one of $R_3$ and $R_4$ is other than H,

and pharmaceutically acceptable salts thereof.

$R_1$ is H or the $-\overset{O}{\overset{\|}{C}}-L$ group, with H being preferred. The L group includes $C_1$-$C_{10}$, linear and branched hydrocarbon alkyl such as methyl, n-decyl, <u>tert</u> butyl, isoamyl, n-heptyl and the like with $C_1$-$C_4$ alkyl being preferred, and phenyl or mono- and disubstituted phenyl such

as <u>tert</u> butylphenyl, 2,6-dibromophenyl, 3-methylphenyl, 4-n-propylphenyl, 3,5-dimethoxyphenyl, 4-iodophenyl, 2-methyl-4-chlorophenyl, 4-fluorophenyl and the like, with monosubstituted phenyl preferred.

The $R_2$ ester groups are $C_1$-$C_6$alkylester exemplified by $-COOCH_3$, $-COOC_6H_{13}$, $-COOCH(CH_3)_2$, $-COOC_2H_5$ and the like and $C_6$-$C_{12}$ carbocyclic aryl ester exemplified by $C_6H_5$-OOC, p-$CH_3$-$C_6H_4$-OOC-, $C_6H_5$-$C_6H_4$-OOC-, $C_{10}H_7$-OOC- and the like; and the amide groups include $-CONH_2$, $C_1$-$C_6$ substituted amide groups such as $-CON(CH_3)_2$, $-CON(C_6H_{13})_2$, $-CONHC_2H_5$, -CON (<u>sec</u> butyl)$_2$ and the like and carbonyl heterocyclic groups such as

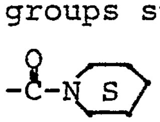 , 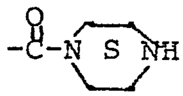 and 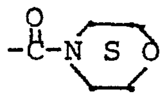 .

The $R_3$/$R_4$ $C_1$-$C_4$alkyl substituent include the branched and unbranched hydrocarbon moieties e.g. $CH_3$, isopropyl, n-propyl etc.; the substituted phenyl groups include mono or disubstituted phenyls such as p-tolyl, 2,6-dibromophenyl, 4-isopropylphenyl, 3,5-dimethoxyphenyl, 2-fluorophenyl, 2-methyl-4-chlorophenyl, 3-chlorophenyl, 4-iodophenyl and the like, monosubstituted phenyl being preferred; and the $C_1$-$C_4$alkyl-CONH groups include $CH_3CONH$, $(CH_3)_3$-C-CONH, $C_2H_5$-CONH and the like.

R is isopropyl, <u>sec</u> butyl, or <u>tert</u> butyl with <u>tert</u> butyl being preferred.

The alkylene moiety bridging positions 4-5 in Formula II may have from 3-6 units. The $-CH_2-CH_2-CH_2-$ and $-CH_2-(CH_2)_2-CH_2-$ groups are preferred.

Examples of compounds of Formula I are

2-(3-<u>tert</u>-butylamino-2-hydroxypropoxy)-3-trifluoromethyl-4-methylpyridine,

2-(3-isopropylamino-2-hydroxypropoxy)-3-cyano-5-acetamidopyridine,

2-(3-tert butylamino-2-hydroxypropoxy)-3-trifluoromethyl-5-methylpyridine,

2-(3-sec butylamino-2-hydroxypropoxy)-3-(1-pyrrolyl)-5-trifluoromethyl pyridine,

2-(3-R-amino-2-$OR_1$-propoxy)-3-cyano-4-phenyl-5-methylpyridine,

2-(3-tert butyl-2-hydroxypropoxy)-3-cyano-4-phenylpyridine and the like.

Examples of Formula II compounds are

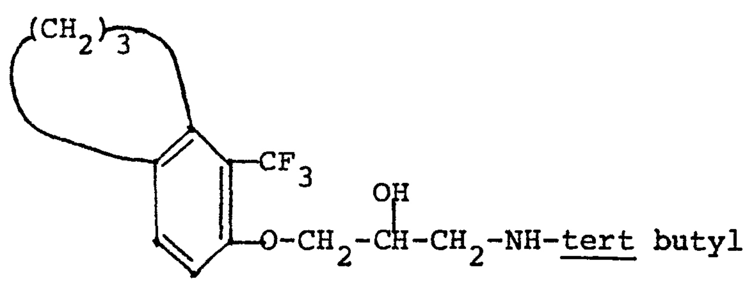

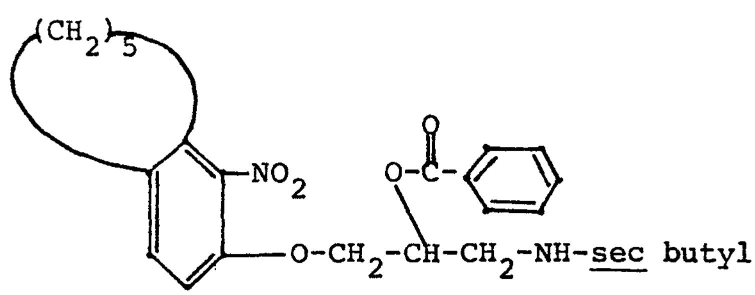

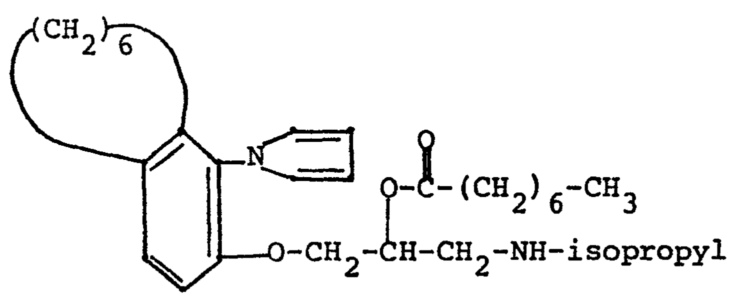

and the like.

A class of preferred compounds are those having the formula

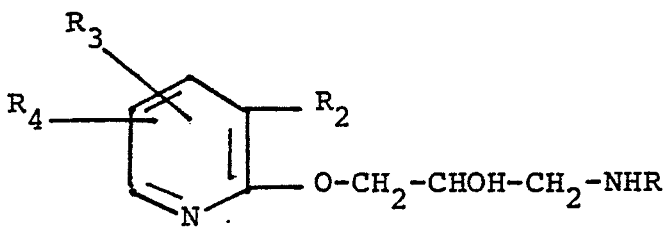

III

Another class of preferred compounds are those of Formula I where $R_4$ is H. These compounds have the formula

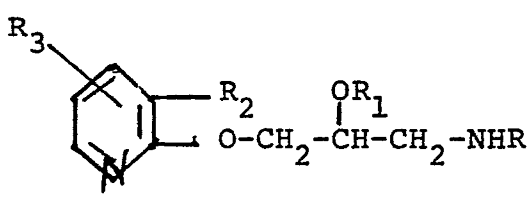

IV

where R, $R_1$, $R_2$ and $R_3$ are as defined above. Preferred $R_3$ substituents are CN, $CF_3$, 1-pyrrolyl, Cl, F, phenyl, $CH_3$ or $C_2H_5$. The compounds of Formula IV where $R_3$ is in the 4 or 5 position are more preferred and where $R_1$ is H and R is tert butyl, the compounds are more particularly preferred. Especially preferred compounds of Formula IV are those wherein additionally $R_2$ is -CN, F or $CF_3$ and most preferably CN or F.

Examples of the compounds of Formula IV are

2-(3-sec butylamino-2-hydroxypropoxy)-3-cyano-4-ethylpyridine,

2-(3-isopropylamino-2-hydroxypropoxy)-3-fluoro-4-methylpyridine,

2-(3-*tert* butylamino-2-hydroxypropoxy)-3-trifluoromethyl-4-fluoropyridine,

2-(3-isopropylamino-2-benzoyloxypropoxy)-3-(1-pyrrolyl)-5-methylpyridine,

2-[3-*sec* butylamino-2-(p-chlorobenzoyloxy)propoxy]-3-cyano-5-(1-pyrrolyl)pyridine,

2-(3-*tert* butylamino-2-hydroxypropoxy)-3-fluoro-4-phenylpyridine,

2-(3-isopropylamino-2-undecanoyloxypropoxy)-3-trifluoromethyl-5-ethylpyridine,

2-[3-*tert* butylamino-2-(p-methoxybenzoyloxy)propoxy]-3-fluoro-4-trifluoromethylpyridine,

2-[3-*sec* butylamino-2-(m-chlorobenzoyloxy)propoxy]-3-cyano-4-fluoropyridine,

2-[3-isopropylamino-2-(2-bromo-4-methylbenzoyloxy)-propoxy]-3-cyano-5-(1-pyrrolyl)pyridine,

2-[3-*tert* butylamino-2-(3,5-dimethoxybenzoyloxy)-propoxy]-3-trifluoromethyl-5-fluoropyridine,

2-(3-*tert* butylamino-2-hydroxypropoxy)-3-fluoro-4-ethylpyridine,

2-(3-isopropylamino-2-octanoyloxypropoxy)-3-fluoro-5-trifluoromethylpyridine,

2-(3-*tert* butylamino-2-isovaleryloxypropoxy)-3-chloro-5-trifluoromethylpyridine

and the like.

Another class of preferred compounds are those of Formula II. More preferred Formula II compounds are those wherein n is 3 or 4, more preferably when $R_2$ is CN or F, and most preferably when $R_2$ is CN, $R_1$ is H and R is *tert* butyl.

The substituted pyridines of the present invention include all the optical isomer forms, that is mixtures of enantiomers e.g. racemates as well as the individual enantiomers. These individual enantiomers are commonly designated according to the optical rotation they effect, by (+) and (-), (L) and (D), (l) and (d) or combinations of these symbols. The symbols (S) and (R) stand for sinister and rectus respectively and designate an absolute spatial configuration of the enantiomer. The (S) isomer is a preferred isomer configuration.

The pyridines of the present invention can be prepared by any convenient process.

One such process involves the coupling of a halopyridine with a suitable substituted oxazolidine and hydrolyzing the reaction product obtained. This process is illustrated by the following set of reaction equations:

Reaction A

(1)

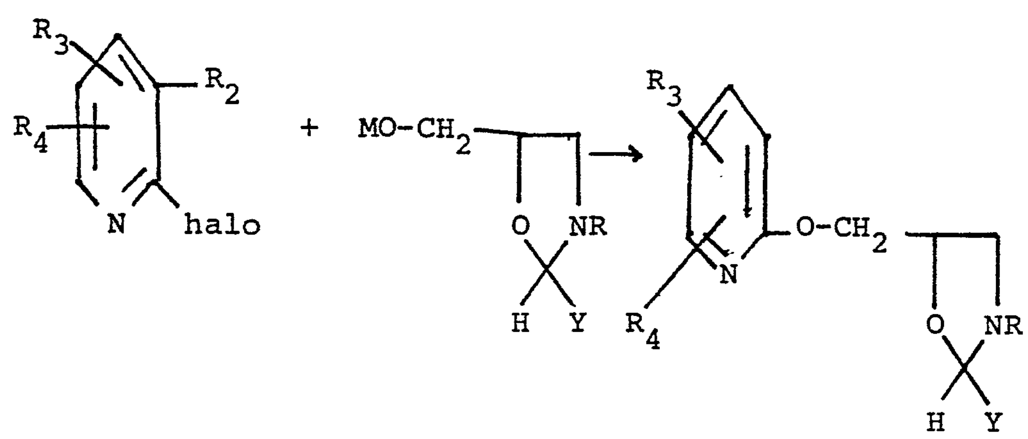

V VI VII

(2)

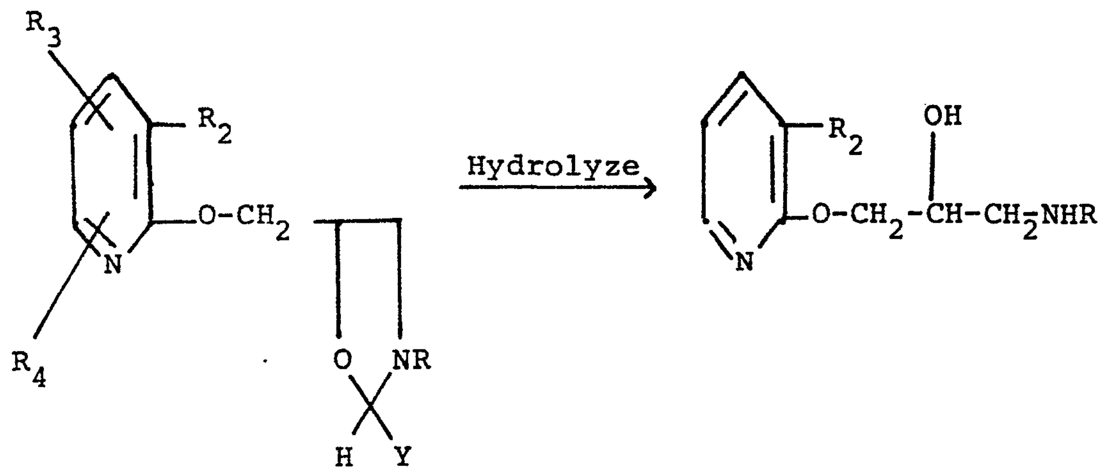


I

Halo may be Cl, Br and I, with Cl being preferred. M is an alkali metal, either potassium or sodium. Y can be hydrogen or the residue of any suitable aldehyde 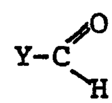 e.g. an arylaldehyde, such as benzaldehyde, naphthaldehyde and the like, or an alkanal such as acetaldehyde, butyraldehyde and the like. The process for preparing oxazolidines where M is hydrogen is disclosed in U.S. 3,718,647 and U.S. 3,657,237 and to the extent necessary the pertinent disclosure is incorporated herein by reference. The alkali metal salt of the oxazolidine is prepared in a conventional manner by reaction of the corresponding hydroxymethyloxazolidine with an appropriate amount of an alkali base reactant. However, this Reaction A may also be carried out with in-situ formation of the

alkali metal oxazolidine salt (Formula VI) by reacting the oxazolidine

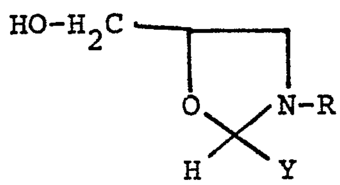

VIII

with the Formula V pyridine in the presence of a strong base such as an alkali metal alkoxide (e.g. $K\text{-}O\text{-}C\text{-}(CH_3)_3$) or sodium hydride.

The coupling reaction can be carried out at temperatures ranging from about 0°C to the reflux temperature of the solvent. A temperature range of about 10°C to about 75°C is preferred. The reaction is generally carried out in a solvent. Any suitable solvent may be used. Examples of useful solvents are dimethylformamide, dimethylsufoxide, hexamethylphosphoramide, tert butanol, alkanols and the like. The hydrolysis is carried out using conventional acid hydrolysis reagent and techniques e.g. treatment with a solution of an acid such as acetic acid or any strong mineral acid such as HCl or $H_2SO_4$. The hydrolysis product can be directly obtained as the salt of the acid used for the hydrolysis. Ordinarily, the product I is recovered as the free base after conventional neutralization of the salt.

The coupling reaction is ordinarily carried out at atmospheric pressure. Higher pressures may be used if desired.

When a racemic oxazolidine (Formula VI or VIII) is used as a reactant, the product is obtained as a racemate. The racemate may be separated into its individual enantiomers by conventional resolution techniques.

When Y in the oxazolidine e.g. (Formula VI or VIII) is other than hydrogen, in addition to the chiral center at oxazolidine position 5 there is a second chiral center at position 2. However, whenever the oxazolidine is designated e.g. as (S), (R) or (R,S), this designation refers only to the optical configuration around the carbon atom at the 5 position.

By using a single optical isomer of said oxazolidine in the above reactions, the product may be obtained directly as a single enantiomer. Thus, if the S-isomer of the oxazolidine is used, then the product obtained will be the S-isomer. This provides a convenient way for directly preparing individual isomers of the present pyridines.

The intermediates of Formula V may be prepared by any conventional process. Especially useful processes involve the condensation of alkylidènemalononitriles or alkylidinecyanoacetates with either $HC(OC_2H_5)_3$ or dimethylformamide dimethylacetal $(H{-}C\begin{smallmatrix}\nearrow (OCH_3)_2 \\ \searrow N(CH_3)_2\end{smallmatrix})$ and cyclizing the unsaturated intermediate product with acid to provide Formula V compounds. These especially useful processes are an extension of the processes described by Bryson et al in J. Org. Chem. 41 2066 (1976) and J. Org. Chem. 39, 3436 (1974). The aforesaid malononitriles and cyanoacetates are prepared by methods available in the literature.

The following reaction equations illustrate the condensation reactions:

METHOD A

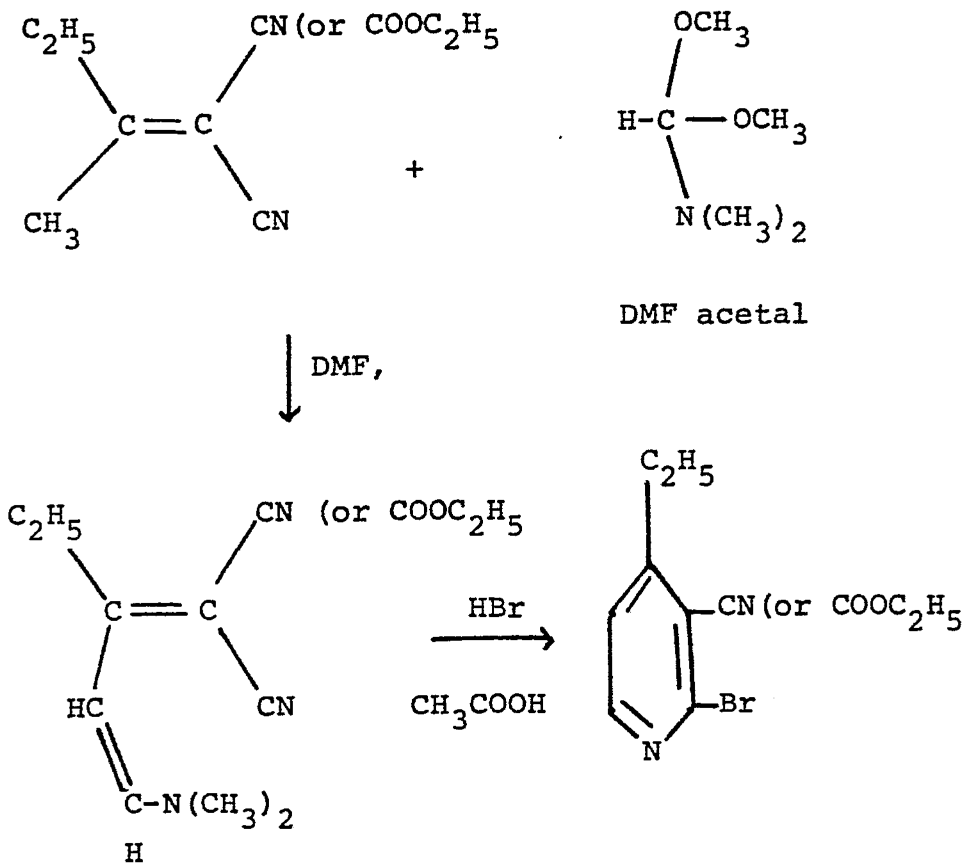


METHOD B

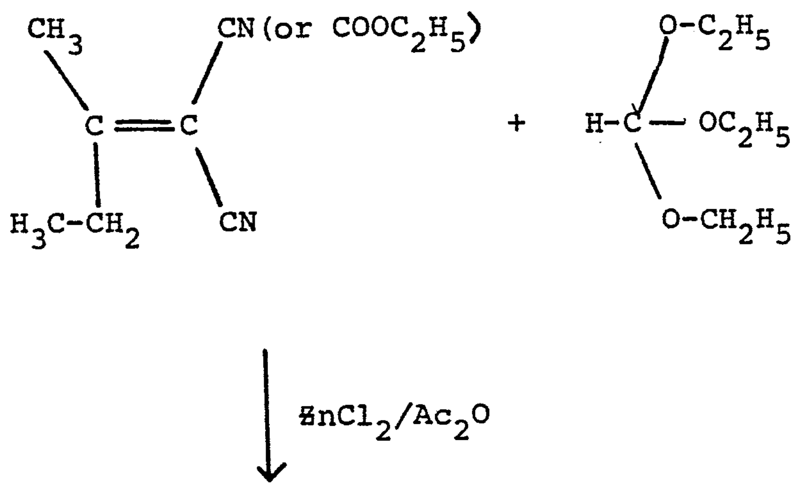

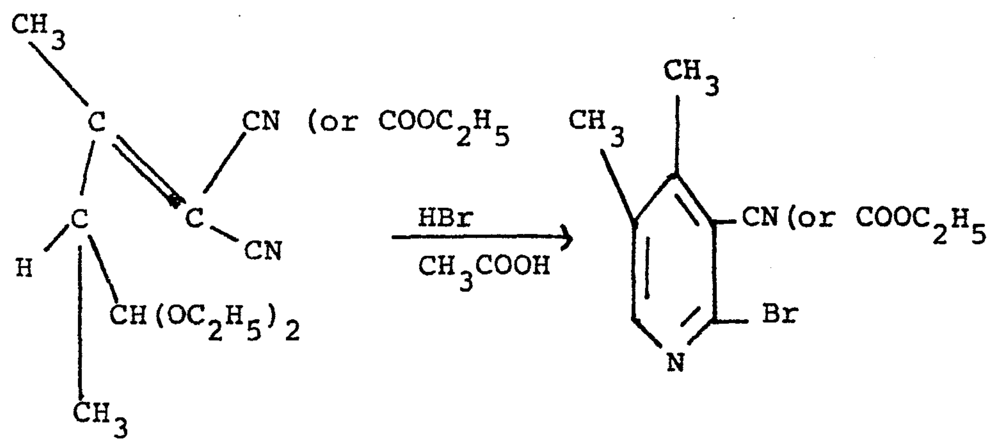


The intermediate pyridines of Formula V having a $CF_3$ substituent are obtained by hydrolyzing the ester group to the acid (COOH) and treating this derivative with a fluorinating agent e.g. $SF_4/HF$ to obtain the $CF_3$ group.

Pyridines of the present invention wherein $R_1$ is other than hydrogen are conveniently prepared by treating the corresponding pyridine where $R_1$ is hydrogen with an appropriate acylating agent such as an acyl halide, e.g. undecanoyl chloride, pivaloyl chloride, benzoylchloride, p-methoxybenzoyl chloride, an anhydride e.g. acetic anhydride, and the like. The reaction is illustrated by the following equations:

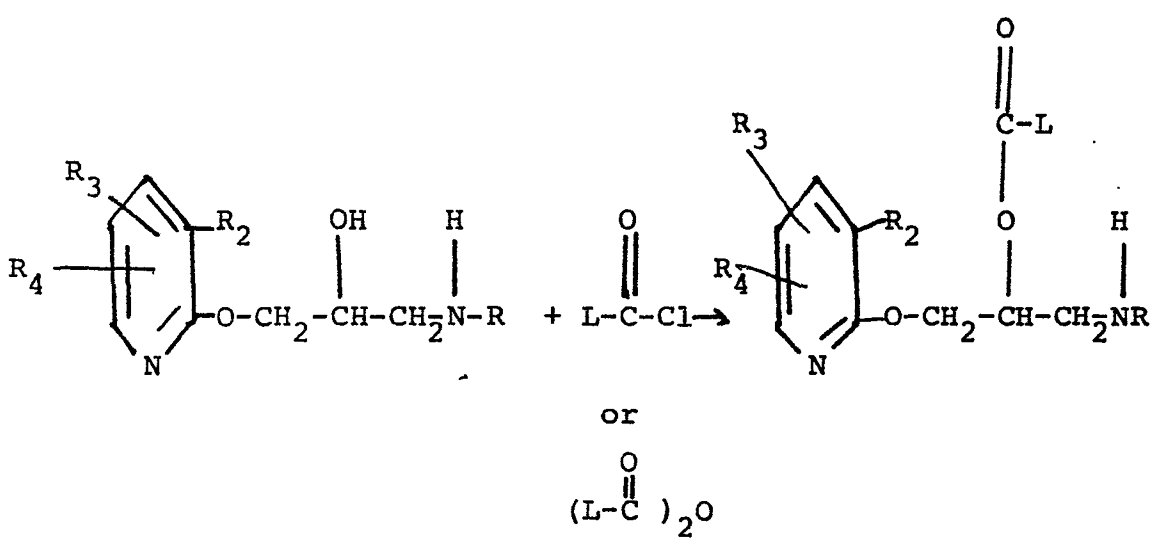

The compounds of the present invention also include the pharmaceutically acceptable salts of the novel pyridines. These salts are generally salts of the Formula I or II pyridines and organic or inorganic acids. These salts are prepared by treating the pyridine with an appropriate amount of a useful acid, generally in a suitable solvent. Examples of useful organic acids are isethionic acid and carboxylic acids such as maleic acid, acetic acid, tartaric acid, propionic acid, fumaric acid, succinic acid, pamoic acid, oxalic acid, pivalic acid and the like; useful inorganic acids are hydrohalo acids such as HCl, HBr, HI; sulfuric acid, phosphoric acid and the like. The hydrochloride and hydrogen maleate salts are examples of preferred salts.

The compounds of the present invention have β-adrenergic blocking activity. This activity is useful in treating cardiovascular related disorders such as angina pectoris, arrhythmia, tachychardia etc. This β-adrenergic blocking activity may also be manifested in an antihypertensive effect of gradual onset after extended administration of the pyridine compound.

The β-adrenergic blocking activity of the present pyridine is determined by measuring the ability of representative pyridines to block the β-adrenergic stimulant effect of isoproterenol.

Some of the present pyridines also exhibit antihypertensive activity of immediate onset This antihypertensive activity is believed to be the result of peripheral vasodilation via a mechanism not directly related to β-adrenergic blockade.

This rapid onset antihypertensive activity is determined by administering a representative

pyridine of the present invention to spontaneously hypertensive (SH) rats and measuring the effect on blood pressure. Examples of representative compounds having this immediate onset antihypertensive activity are (S) 2-(3-tert butylamino-2-hydroxypropoxy)-3-fluoro-5-trifluoromethylpyridine, (S) 2-(3-tert butylamino-2-hydroxypropoxy)-3-cyano-5-(1-pyrrolyl)-pyridine, (S) 2-(3-tert butylamino-2-hydroxypropoxy)-3-cyano-5-methylpyridine and (S) 2-(3-tert butylamino-2-hydroxypropoxy)-3-trifluoromethyl-5-fluoropyridine.

The observed β-adrenergic blocking activity of the present pyridines in test animals indicates that they are useful in humans as β-adrenergic blocking agents. The ability of certain of the present pyridines to reduce blood pressure, in an SH rat, rapidly and for extended duration, also indicates that the present pyridines are useful to treat hypertension in humans.

For use as β-adrenergic blocking agents, and/or antihypertensive agents the compounds of the present invention can be administered orally, by inhalation, by suppository or parenterally i.e. intravenously, intraperitoneally, etc. and in any suitable dosage form. The compounds may be offered in a form (1) for oral administration e.g. as tablets in combination with other compounding ingredients (diluents or carriers) customarily used such as talc, vegetable oils, polyols, benzyl alcohols, starches, gelatin and the like - or dissolved, dispersed or emulsified in a suitable liquid carrier - or in capsules or encapsulated in a suitable encapsulating material; or (2) for parenteral administration, dissolved, dispersed, or emulsified in a suitable liquid carrier or diluent or (3) as an aerosol or (4) as a suppository. The ratio of active

ingredient (present pyridine) to compounding ingredients will vary as the dosage form requires. Conventional procedures are used to prepare the pharmaceutical formulations.

The dosage level for the present compounds may be varied from about 0.01 mg. to about 50 mg. per kilogram of animal body weight per day. Daily doses ranging from about 0.04 to about 2.5 mg/kg are preferred, with about 0.08 to about 1.25 mg/kg being a more preferred range. Oral administration is preferred. Either single or multiple daily doses may be administered depending on unit dosage.

Thus, another embodiment of this invention is a pharmaceutical composition containing β-adrenergic blocking or antihypertensive amount of a compound of the present invention.

The following examples illustrate the Method A and B preparations and conversion of the ester to the $CF_3$ derivative. Temperatures are in ° Celsius.

## EXAMPLE 1

### METHOD A - Preparation of 2-Bromo-3-trifluoromethyl-5-methylpyridine

a. To a solution of ethylpropylidene cyanoacetate (11.4 g, 0.074 m) in absolute ethanol (75 ml) was added DMF acetal (8.9 g, 0.074 m). After the addition, the solution was heated at reflux for 6 hours and then concentrated to dryness to yield 16.0 grams of crude 2-cyano-5-(N,N-dimethylamino)-4-methyl-2,4-pentanedienoate.

b. The crude 2-cyano-5-(N,N-dimethylamino)-4-methyl-2,4-pentanedienoate (15.9 g) was dissolved in acetic acid (50 ml) and the mixture heated at 40°. A solution of 30% HBr/acetic acid (100 ml) was added dropwise and then the mixture was heated to 55° with stirring. After heating for 3/4 of an hour, the solution was poured onto ice, neutralized with solid $Na_2CO_3$ and extracted with $CH_2Cl_2$ (4X200 ml). The organic extracts were dried over $Na_2SO_4$, filtered and concentrated to dryness. The residue was distilled at 114-120° (0.4 mm) to yield 6.3 g (35% yield) of ethyl 2-bromo-5-methylnicotinate

c. A mixture of ethyl 2-bromo-5-methylnicotinate (7.1 g, 0.03 m) and 10% NaOH solution (500 ml) was heated on a steam bath with stirring. After 3 hours, the solution was cooled and neutralized with 12N HCl. After cooling in an ice bath, the mixture was filtered to yield 5.6 g (39% yield) of 2-bromo-5-methylnicotinic acid.

The 2-bromo-5-methylnicotinic acid (5.0 g, 0.023m), $SF_4$ (31 g; 0.020 m) and HF (5.3 ml) were charged to a steel bomb. The contents were heated at 120° for 8 hours. After cooling to room temperature, the bomb was opened and the contents poured into saturated $Na_2CO_3$ solution

and extracted with $CHCl_3$ (3X100 ml). The organic layer was dried over $Na_2SO_4$, filtered and concentrated to dryness. The residue distilled at 45-49° (0.1 mm) to yield 4 g (59% yield) of 2-bromo-3-trifluoromethyl-5-methylpyridine.

EXAMPLE 2

METHOD B - Preparation of 2-Bromo-3-cyano-5-methylpyridine

a. A mixture of 1-propylidenemalononitrile (11.3 g, 0.104 m), acetic anhydride (21 ml), $HC(OEt)_3$ (16.3 g, 0.11 m) and $ZnCl_2$ (100 mg) was heated overnight at 145°. After 18 hours, the volatiles were removed by distillation at atmospheric pressure, acetic anhydride (5 ml) and $HC(OC_2H_5)_3$ (4 ml.) were added and the mixture heated at 150°. After 10 hours, the solution was cooled and added to saturated $Na_2CO_3$ solution. The aqueous solution was extracted with $CHCl_3$ (3X100 ml). The organic layer was dried over $Na_2SO_4$, filtered and concentrated to dryness. The residue distilled at 138-158° (0.3 mm) to give 11.2 g (67% yield) of 1,1-dicyano-4-ethoxy-3-methyl-1,3-butadiene.

b. 1,1-Dicyano-4-ethoxy-3-methyl-1,3-butadiene (11.2 g) was cyclized using basically the same procedure as Example 1 b.; and 3.0 g (15% yield) of 2-bromo-3-cyano-5-methylpyridine, m.p. = 109°-111°, were obtained.

The following table lists the Formula V intermediates which were prepared using the processes substantially as disclosed in Examples 1 and/or 2, as applicable.

TABLE 1

| Ex. | Process of Example | Starting Material | Formula V Product | Product Yield wgt -% | Product M.P. or B.P. (mm.Hg) |
|---|---|---|---|---|---|
| 3 | 1a)b)c) | 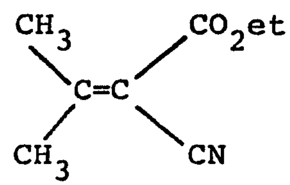 | 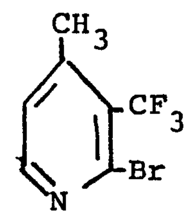 | 5.2g-58% | 110-112°C (at 0.7) |
| 4 | 1a)b) | 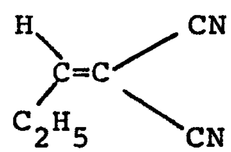 | 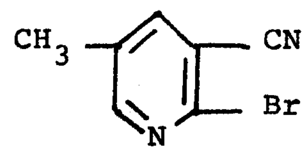 | 0.4g-5% | 109°-111°C |
| 5 | 1a)b) | 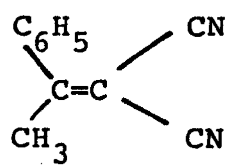 | 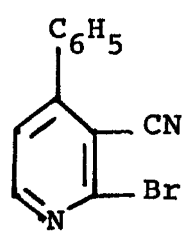 | 3.8g-22% | 122°-125°C |

TABLE 1 (Cont'd.)

| Ex. | Process of Example | Starting Material | Formula V Product | Product Yield wgt - % | Product M.P. or B.P. (mm.Hg) |
|---|---|---|---|---|---|
| 6 | 1a)b) | 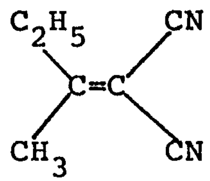 | 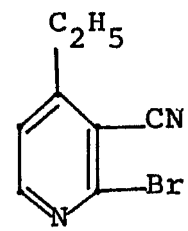 | 2.4g-16% | 64°-65°C |
| 7 | 1a)b) | 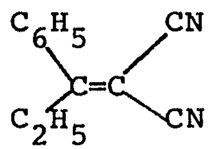 | 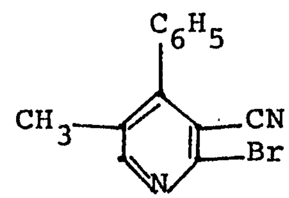 | 0.15g-3% | 123°-124°C |
| 8 | 2a)b) | 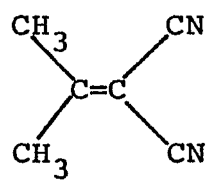 | 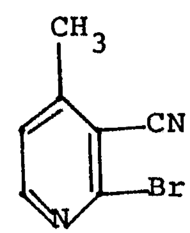 | 1.0 g-23% | 109°-111°C |

TABLE 1 (Cont'd.)

| Ex. | Process of Example | Starting Material | Formula V Product | Product Yield wgt - % | Product M.P. or B.P. (mm.Hg) |
|---|---|---|---|---|---|
| 9 | 2a)b) | 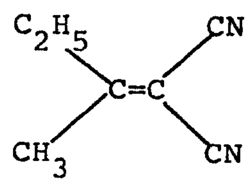 | 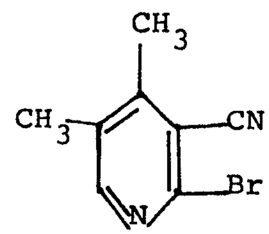 | 6.3g-29% | 93°-95°C |
| 10 | 2a)b) | 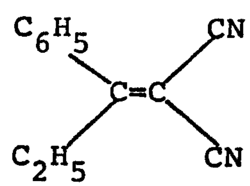 | 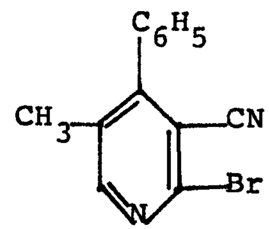 | 5.7g-42% | 123°-124°C |
| 11 | 2a)b) | 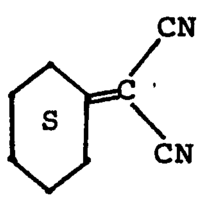 | 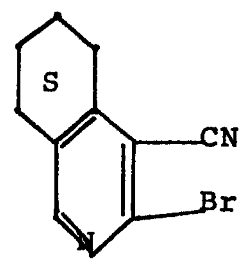 | 2.0g-15% | 128.5°-130.5°C |

TABLE 1 (Cont'd.)

| Ex. | Process of Example | Starting Material | Formula V Product | Product Yield wgt - % | Product M.P. or B.P. (mm.Hg) |
|---|---|---|---|---|---|
| 12 | 2a)b) | 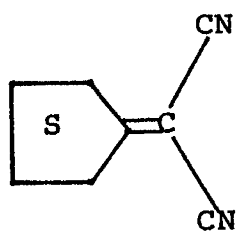 | 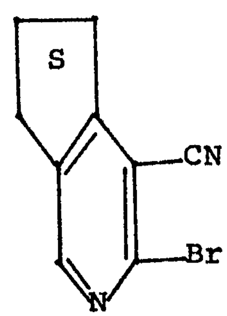 | 3.4g-15% | 103°-105°C |
| 13 | 2a)b) | 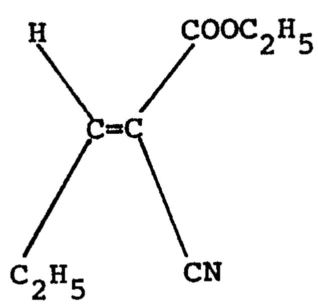 | 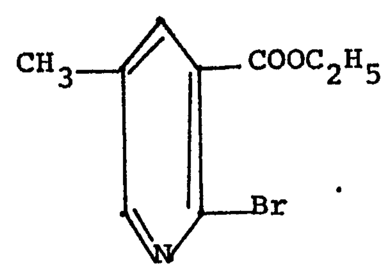 | 4.3g-40$ | 114°-120°C(0.4) |

The following examples illustrate the preparation of other intermediates of Formula V. Temperatures are °C.

EXAMPLE 14

5-Acetamido-2-Chloronicotinonitrile

A solution of 5-amino-2-chloronicotinonitrile (2.6 g, 0.017 m) and acetic anhydride (50 ml) was heated at reflux for 30 minutes. After cooling to 25°, the reaction mixture was poured into water, neutralized with saturated $Na_2CO_3$ solution and extracted with $CH_2Cl_2$. The organic layer was dried over $Na_2SO_4$, filtered and concentrated to dryness. The residue was recrystallized from benzene/hexane to yield 2.5 g (76% yield) of 5-acetamido 2-chloronicotinonitrile.

Substituting appropriate alkanoic acid anhydrides for acetic anhydride in Example 14, the corresponding alkanoylamino substituted nicotinonitrile is obtained.

EXAMPLE 15

2-Chloro-5-(1-pyrrolyl)nicotinonitrile

A solution of 5-amino-2-chloronicotinonitrile (5.0 g, 0.033 m), 2,5-dimethoxytetrahydrofuran (11 g, 0.085 m) and acetic anhydride (20 ml) was heated at reflux with stirring. After two hours, the solution was concentrated to dryness under reduced pressure (20 mm). The residue was triturated with $CCl_4$ and filtered to yield 5.2 g (78% yield) of 2-chloro-5-(1-pyrrolyl)nicotinonitrile, m.p. = 144°-146°.

EXAMPLE 16

2-Fluoro-3-nitro-5-trifluoromethylpyridine/2-chloro-3-nitro-5-trifluoromethylpyridine mixture

A steel bomb was charged with 6-chloro-5-nitronicotinic acid (4.1 g, 0.02 m), $SF_4$ (21 g) and HF (3.7 ml). The mixture was heated at 90° for eight hours. After cooling to 25°, the bomb was vented and the contents poured onto ice. The solution was neutralized with saturated $Na_2CO_3$ solution and extracted with $CH_2Cl_2$. The organic layer was dried over $Na_2SO_4$, filtered and the solution was distilled. A mixture of 2-chloro and 2-fluoro-3-nitro-5-trifluoromethylpyridine (3.3 g) was obtained by distillation at 198°-200° (760 mm).

## EXAMPLE 17

### 2-Fluoro-3-amino-5-trifluoromethylpyridine/2-chloro-3-amino-5-trifluoromethylpyridine mixture

a. To a solution of 6-chloro-5-nitronicotinic acid in acetic acid (240 ml) was added iron (20 g) and the mixture was heated with stirring on a steam bath. After 1 1/2 hours, the mixture was filtered hot and washed with hot acetic acid. The filtrate was concentrated to dryness and the residue was treated with 10% NaOH, filtered and the pH adjusted to 2-3. The solid was filtered to yield 8.0 g (60% yield) of 5-amino-6-chloronicotinic acid.

b. A steel bomb was charged with 5-amino-6-chloronicotinic acid (8.0 g, 0.046 m), $SF_4$ (12 g) and HF (4.2 g). The mixture was heated at 120° for eight hours. After cooling to 25°, the bomb was vented and the contents poured onto ice. The solution was neutralized with saturated $Na_2CO_3$ and extracted with $CHCl_3$. The organic layer was dried over $Na_2SO_4$, filtered and concentrated to dryness. The residue was sublimed at 50°-65° at 1.4 mm to yield 4.4 g of a mixture of 2-chloro and 2-fluoro-3-amino-5-trifluoromethylpyridine.

## EXAMPLE 18

### 2-Fluoro-3-(1-pyrrolyl)-5-trifluoromethylpyridine/2-Chloro-3-(1-pyrrolyl)-5-trifluoromethylpyridine mixture

A solution of the mixture (1.1 g) from Example

17 b., 2,5-dimethoxyltetrahydrofuran (0.9 g, 0.007 m) and acetic acid (5 ml) was heated at reflux with stirring. After 1 1/2 hours, the solution was cooled and poured into saturated $Na_2CO_3$ solution. The aqueous layer was extracted with $CHCl_3$ and the organic layer was dried over $Na_2SO_4$, filtered and concentrated to dryness. The residue was chromatographed on silica gel and the product eluted with 1:1 $CH_2Cl_2$:hexane to yield 0.65 g of a mixture of 2-chloro and 2-fluoro-3-(1-pyrrolyl)-5-trifluoromethylpyridine.

## EXAMPLE 19

2-3-fluoro-5-trifluoromethylpyridine

A steel bomb was charged with 6-chloro-5-fluoronicotinic acid (5.4 g, 0.03 m), $SF_4$ (32.4 g) and HF (5.6 ml). The mixture was heated at 120° for eight hours. After cooling to 25°, the bomb was vented and the contents poured onto ice. The solution was neutralized with saturated $Na_2CO_3$ solution and extracted with $CH_2Cl_2$. The organic layer was dried over $Na_2SO_4$, filtered and the solvent distilled off on a steam bath. The residue was distilled at 115° (760 mm) to yield 1.4 g (23% yield) of 2-chloro-3-fluoro-5-trifluoromethylpyridine.

## EXAMPLE 20

2-Chloro-5-fluoro-3-trifluoromethylpyridine

A steel bomb was charged with 2-chloro-5-fluoronicotinic acid (4.5 g, 0.026 m), $SF_4$ (27 g) and HF (4.7 ml). The mixture was heated at 120° for eight hours. After cooling to 25°, the bomb was vented and the contents poured onto ice. The solution was neutralized with saturated $Na_2CO_3$ solution and extracted with $CH_2Cl_2$. The organic layer was dried over $Na_2SO_4$, filtered and the solvent distilled off on a steam bath. The residue was distilled at 125° (760 mm) to yield 3.2 g (23% yield) of 2-chloro-5-fluoro-3-trifluoromethylpyridine.

EXAMPLE 21

1-Propylidenemalononitrile

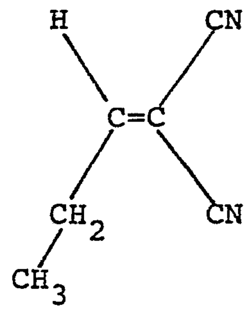

1-Propylidenemalononitrile (61.7 g b.p. = 45 at 0.6 mm) was obtained from the reaction of malononitrile (50 g), propionaldehyde (47 g) acetic acid (10 ml), alanine (0.5 g) and benzene (140 ml) using the general procedure described by F.S. Prout in J. Org. Chem. 18, 928 (1953).

The following examples illustrate the preparation of the compounds of Formulae I and II. All temperatures are in °C.

EXAMPLE 22

(S) 4-Methyl-2-(3-*tert* butylamino-2-hydroxypropoxy)-3-trifluoromethylpyridine hydrogen maleate

A dry flask was charged, under nitrogen, with (S) 2-phenyl-3-*tert* butyl-5-hydroxymethyloxazolidine (7.0 g, 0.03 m), dimethylformamide (DMF) (80 ml) and NaH (50% mineral oil, 1.3 g, 0.027 m) and heated, with stirring, at 90° for 30 minutes. After cooling to room temperature, 2-bromo-4-methyl-3-trifluoromethylpyridine (6.2 g, 0.026 m) in DMF (25 ml) was added and the mixture was stirred at 45°. After stirring overnight at 45°, the mixture was poured into saturated $Na_2CO_3$ (300 ml) and water (300 ml) and extracted with $Et_2O$ (4X300 ml). The organic layer was washed with water (2X300 ml) and cold 1N HCl (3X200 ml). The acid layer was poured into sodium

acetate $.3H_2O$ (82 g, 0.6 m) and the solution stirred at room temperature. After six hours, the solution was extracted with $Et_2O$ (2X250 ml). The aqueous layer was neutralized with saturated $Na_2CO_3$ and extracted with $CH_2Cl_2$ (4X200 ml). The organic layer was dried over $Na_2SO_4$, filtered and concentrated to dryness. The residue was chromatographed on silica gel 60 and the product eluted with 20% $CH_3OH$-$CHCl_3$. The crude product was crystallized with maleic acid in isopropanol to give 0.8 g (8% yield) of (S) 4-methyl-2-(3-tert butylamino-2-hydroxypropoxy)-3-trifluoromethylpyridine hydrogen maleate, m.p. = 126°-128° ($Et_2O$-isopropanol).

EXAMPLE 23

(S) 3-Cyano-4,5-dimethyl-2-(3-tert butylamino-2-hydroxypropoxy)pyridine hydrogen maleate

A dry flask was charged, under nitrogen, with tert butanol (50 ml), potassium metal (0.58 g, 0.015 m), and (S) 2-phenyl-3-tert butyl-3-hydroxymethyloxazolidine (4 g, 0.017 m) and heated at 40° with stirring until all the potassium metal reacted. Then, 2-bromo-3-cyano-4,5-dimethylpyridine (3.0 g, 0.014 m) in tert butanol (5 ml) was added and the mixture heated at 70°. After heating overnight, the mixture was concentrated to dryness and stirred with water (150 ml) and acetic acid (9.0 g). After five hours, the solution was extracted with $Et_2O$ (2X100 ml). The aqueous layer was neutralized with saturated $Na_2CO_3$ and extracted with $CHCl_3$ (3X100 ml). The organic layer was dried over $Na_2SO_4$, filtered and concentrated to dryness. The residue was crystallized with maleic acid in isopropanol to give 4.1 g (75% yield) of (S) 3-cyano-4,5-dimethyl-2-(3-tert butylamino-2-hydroxypropoxy)pyridine hydrogen maleate, m.p. = 148°-150° ($Et_2O$-isopropanol).

The free base may be obtained from the hydrogen maleate salt by conventional neutralization with an appropriate base. Other salts may be prepared from the free base by treating with an appropriate acid.

The following Table contains additional examples of the Formula I and II pyridines which were prepared using the appropriate Formula V intermediates in a procedure substantially as described in Examples 22 or 23.

TABLE 2

| Ex | Pyridine Intermediate | (S) Isomer Product ① | % Yield | M.P. (°C) |
|---|---|---|---|---|
| 24 | 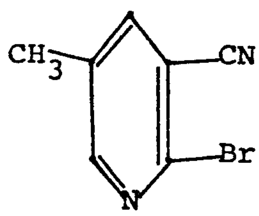 | 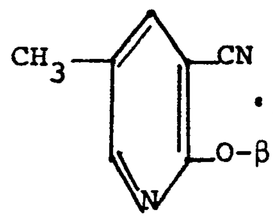 • HMal | 41 | 160-162 |
| 25 | 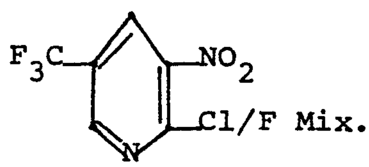 | 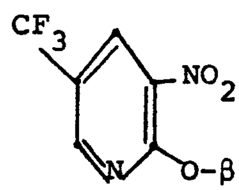 • HCl | 8 | 176-178 |
| 26 | 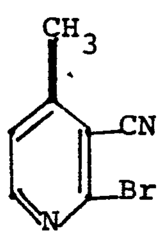 | 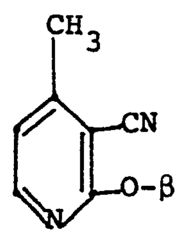 • HMal | 6 | 160-162 |
| 27 | 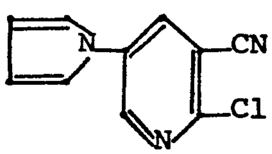 | 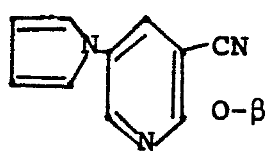 • HCl | 14 | 221-223 |
| 28 | 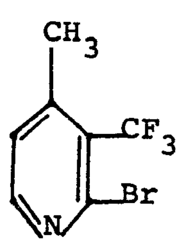 | 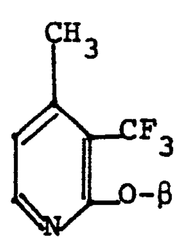 • HMal | 8 | 126-128 |

TABLE 2 (cont.)

| Ex | Pyridine Intermediate (S) (Yield M.P.(°C)) | Isomer Product① | % Yield | M.P.(°C) |
|---|---|---|---|---|
| 29 | 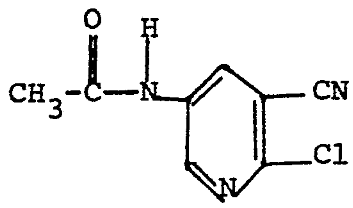 | 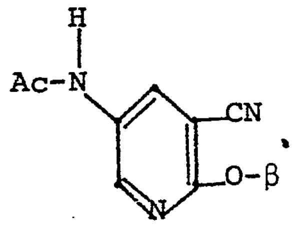 ·HMal | 4.5 | 98-100 |
| 30 | 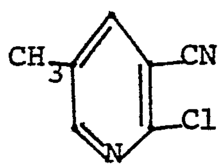 | 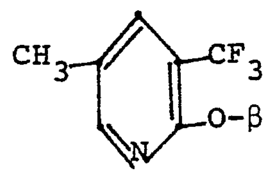 ·HMal | 8.6 | 149-151 |
| 31 | 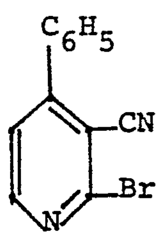 | 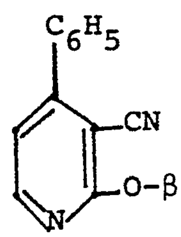 ·HCl | 21 | 193-196 |
| 32 | 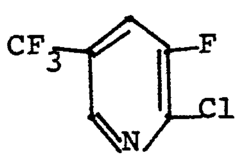 | 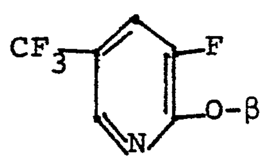 | 50 | 71-73 |
| 33 | 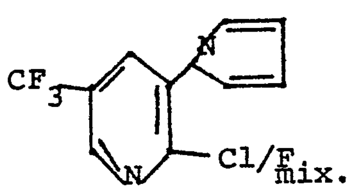 | 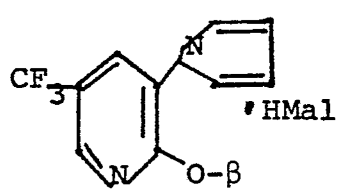 ·HMal | 23 | 129-130 |

TABLE 2 (cont.)

| Ex | Pyridine Intermediate | (S) Isomer Product (1) | % Yield | M.P.(°C) |
|---|---|---|---|---|
| 34 | 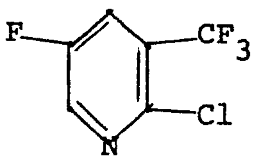 | 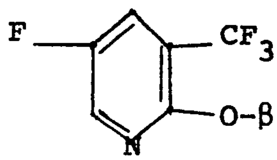 .HMal | 16 | 101-103 |
| 35 | 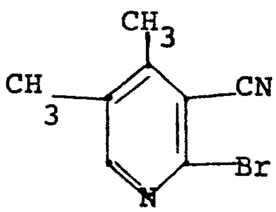 | 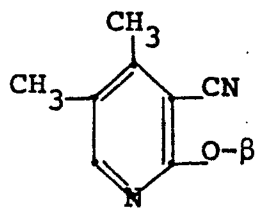 .HMal | 75 | 148-150 |
| 36 | 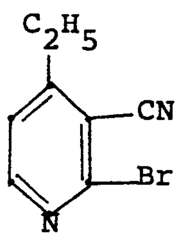 | 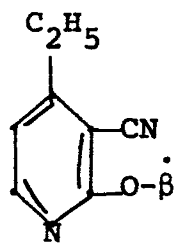 .HMal | 56 | 123-125 |
| 37 | 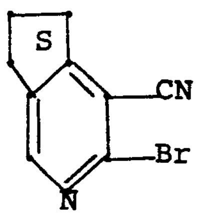 | 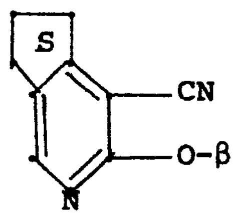 | 35 | 101 |
| 38 | 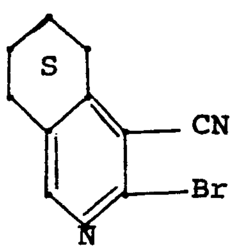 | 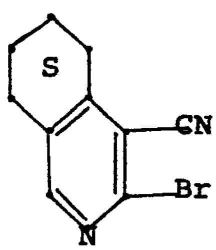 | 53 | 54-56 |

TABLE 2 (cont.)

| Ex | Pyridine Intermediate | (S) ① Isomer Product | | % Yield | M.P.(°C) |
|---|---|---|---|---|---|
| 39 | 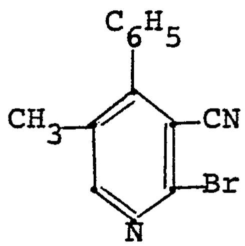 | 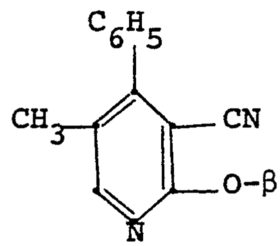 | .HCl | 5 | 243-244 |
| 40 | 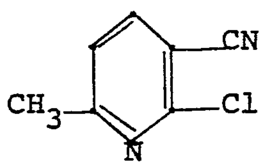 | 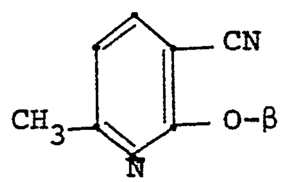 | .HCl | 20 | 208-210 |
| 41 | 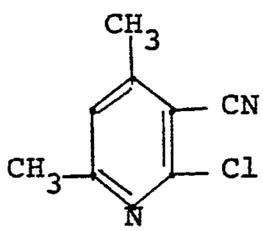 | 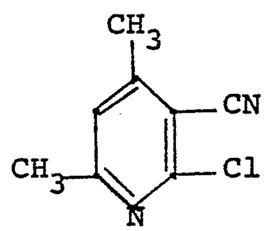 | .HCl. 1/2$H_2O$ | 22 | 177-178 |

① - β = $CH_2$-CHOH-$CH_2$-NH-<u>t</u>-butyl; HMal = hydrogen maleate; HCl = hydrochloride.

While the Examples are directed to (S) isomers, the corresponding (R) isomer or (R/S) racemate is prepared by substituting the appropriate (R) or (R/S) oxazolidine for the (S) oxazolidine in the processes illustrated by Examples 22-39.

Claims to the invention follow.

WHAT IS CLAIMED IS:

1. Compound having the formula

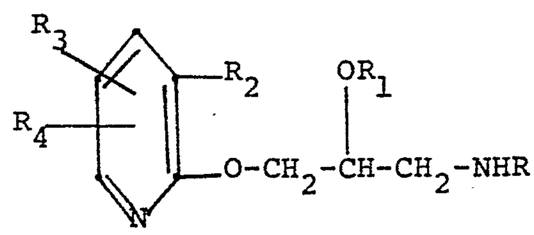

I

or

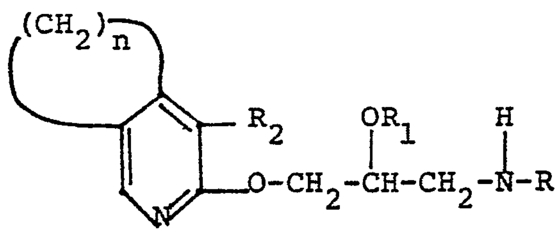

II

wherein

n is 3, 4, 5 or 6,

R is $C_3$-$C_4$ branched alkyl,

$R_1$ is H or $-\overset{O}{\overset{\|}{C}}-L$ wherein L is selected from $C_1$-$C_{10}$ alkyl, phenyl and substituted phenyl having up to two substituents which are independently selected from $C_1$-$C_4$ alkyl, $C_1$-$C_4$ alkoxy and halo,

$R_2$ is F, CN, $CF_3$, Cl, 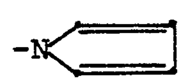 , $NO_2$, $-COOR_5$ wherein $R_5$ is H, $C_1$-$C_6$alkyl or $C_6$-$C_{12}$ carbocyclic aryl,

-CONR$_6$R$_7$ wherein R$_6$ and R$_7$ when separate, are H or $C_1$-$C_6$ alkyl and when joined, are $-CH_2-(CH_2)_3-CH_2-$, $-CH_2-CH_2-O-CH_2-CH_2-$, $-CH_2-CH_2-NH-CH_2-CH_2-$ or $-CH_2-CH_2-N(CH_3)-CH_2-CH_2-$,

and

R$_3$and R$_4$ are independently selected from H, $C_1$-$C_4$alkyl, $CF_3$, F, 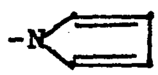, $C_1$-$C_4$alkyl-$\overset{O}{C}$-NH, phenyl and substituted phenyl wherein said substitutents are independently selected from $C_1$-$C_4$alkyl, $C_1$-$C_4$alkoxy and halo, such that one of R$_3$ and R$_4$ is other than H,

and pharmaceutically acceptable salts thereof.

2. Compound of Claim 1 having the S-isomer configuration.

3. Compound of Claim 1, Formula I wherein a) R$_4$ is H, b) R$_4$ is H and R$_1$ is H, c) R$_2$ is CN or F, or d) R$_2$ is CN or F, R$_1$ is H and R is <u>tert</u> butyl.,

4. Compound of Claim 1, Formula I, S-isomer, selected from

(a)

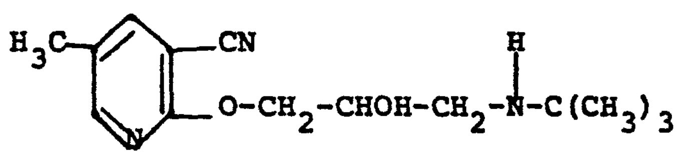

(b)

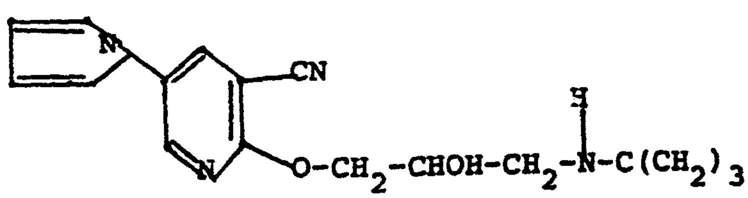

(c)

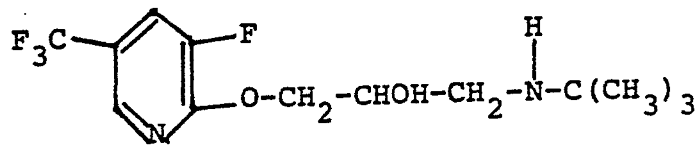

(d)

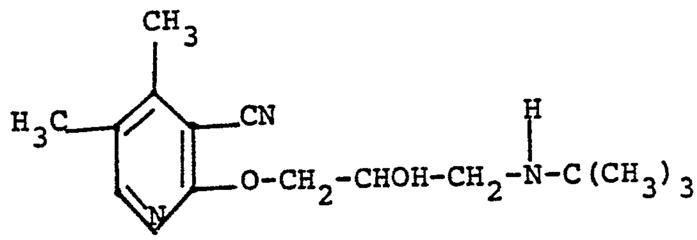

(e)

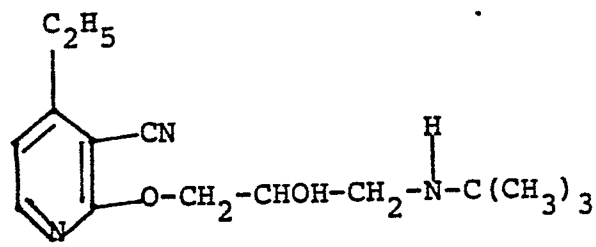

(f)

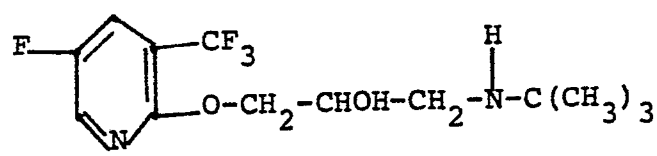

(g)

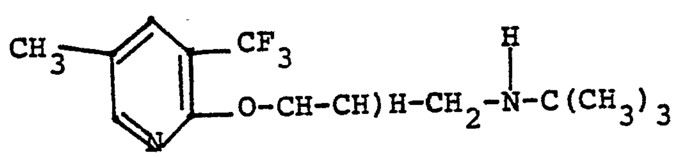

(h)

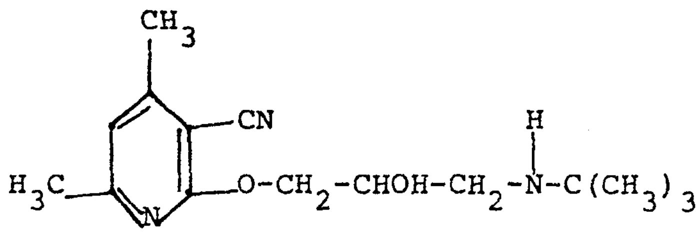

or

(j)

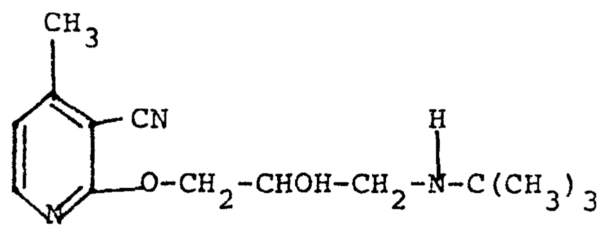

and pharmaceutically acceptable salts thereof.

5. Compound of Claim 1, Formula II.

6. Compounds of Claim 5 wherein n is 3 or 4, $R_2$ is H and R is <u>tert</u> butyl.

7. A pharmaceutical composition for effecting β-adrenergic blockade containing an effective amount of a compound of Claim 1.

8 . A pharmaceutical composition for treating hypertension containing an effective amount of a compound of Claim 4.

9 . A process for preparing compounds having the formula

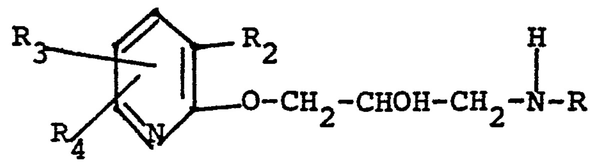 ,

I

or

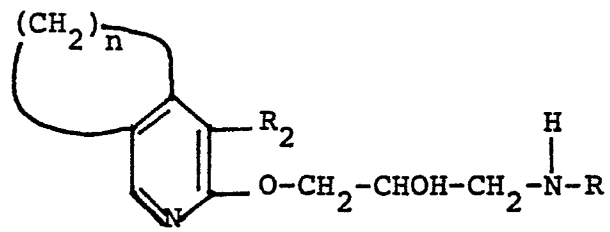

II

which comprises hydrolyzing a compound of the formula

(i)

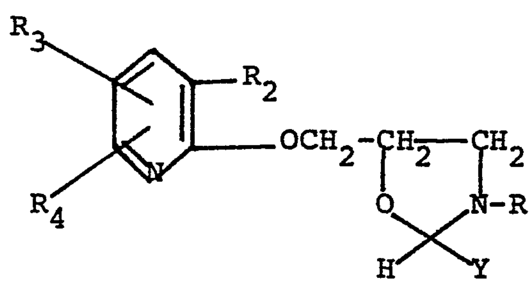

or

(ii)

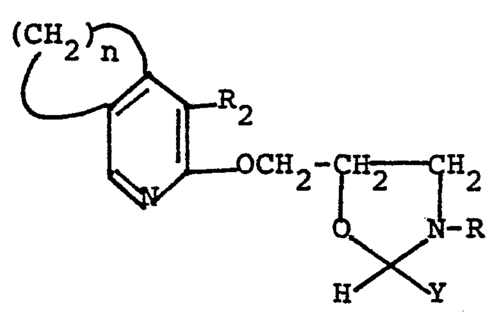

wherein n, R, $R_1$, $R_2$, $R_3$ and $R_4$ are defined as in Claim 1

and Y is an aldehyde residue.

10. The process of Claim 9 wherein said compounds have the (S) isomer configuration.

0003278

European Patent Office

# EUROPEAN SEARCH REPORT

Application number
EP 79 10 0015

## DOCUMENTS CONSIDERED TO BE RELEVANT

| Category | Citation of document with indication, where appropriate, of relevant passages | Relevant to claim |
|---|---|---|
| X | FR - A - 2 218 101 (CIBA-GEIGY)<br>* Pages 1-70 * | 1,2,3,7,8 |
| | -- | |
| | US - A - 4 053 605 (J.J.BALDWIN)<br>* Claims * | 1,7 |
| | -- | |
| | NL - A - 77 05881 (MERCK & CO.)<br>* Claims *<br>& FR - A -2 361 370 | 1,7,9 |
| | -- | |
| | DE - A - 2 507 902 (CIBA-GEIGY)<br>* Claims * | 1,7 |
| | -- | |
| | FR - A - 2 294 702 (MERCK & CO.)<br>* Claims * | 1,7,9 |
| | -- | |
| | FR - A - 2 305 182 (SANDOZ)<br>* Claims 1-4 * | 1,5,7 |
| | ---- | |

CLASSIFICATION OF THE APPLICATION (Int. Cl.²)

C 07 D 213/64
213/85
213/80
401/04
221/04
217/26
217/24
A 61 K 31/44
31/435//
C 07 D 213/61
213/73

TECHNICAL FIELDS SEARCHED (Int.Cl.²)

C 07 D 213/64
213/63
213/85
401/04
217/24
217/26
221/04
A 61 K 31/44
31/435

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 06-04-1979 | VERHULST |

EPO Form 1503.1 06.78